# EUROPEAN PATENT APPLICATION

(11) **EP 3 643 258 A1**
(43) Date of publication of application: **29.04.2020**
(21) Application number: 18201853.1
(22) Date of filing: 22.10.2018
(51) Int. Cl.: A61B 17/17, A61B 17/74, A61B 17/80, A61B 17/88

(54) **IMPLANTATION SYSTEM FOR TREATMENT OF BONE FRACTURES AND HANDLING TOOL FOR AN IMPLANTATION SYSTEM**

(71) Applicant: Stöckli Group AG, 6214 Schenkon (CH)
(72) Inventor: Welte, Thomas, 79576 Weil am Rhein (DE); Schwer, Stefan, 79540 Lörrach (DE)
(74) Representative: Banse & Steglich Patentanwälte PartmbB

(57) **Abstract**

The invention relates to an implantation system for implantation into a bone of a patient, comprising:
- a longitudinal pin implant for inserting into a first bone part of the bone, wherein the pin implant has a front section with one or more groove-like cut-outs extending in axial direction of the front section and opening towards the front end of the implant, wherein at least one of the cut-outs is provided as a guidance cut-out which extends over the full length of the pin implant;
- a bone plate including
∘ an attachment portion for attachment onto an outer surface of a second bone part, and
∘ a guidance sleeve for slidably holding the pin implant and including at least one guidance structure to engage with the at least one guidance cut-outs

## Description

### Technical field

The present invention relates to implantation systems for implanting into a bone particularly for treating bone fractures.

### Technical background

Bone fractures are often treated by implanting medical devices to stabilize the fracture. The fixation of femur fractures is especially demanding due to the high mechanical stress imposed on the femur neck. Particularly, for treatment of femoral neck fractures, a general approach includes the insertion of a pin implant into the femoral head along an axis of the femoral neck and to apply measures to securely connect the pin implant with the femoral head. A proximal end of the pin implant is secured by means of a bone plate on the surface of a femoral shaft. The bone plate is fastened at an outer surface of the femoral shaft so that an arrangement of the femoral shaft and the femoral head is fixed.

Further, any torque applied onto the pin implant by the femoral head is required to be taken up by the bone plate so that the torque can be absorbed in the bone shaft to which the bone plate is attached. Thereby, a rotation of the pin implant is prevented ensuring that the femoral head cannot rotate with respect to the fracture.

Further, to avoid any damages on the hip joint when the pin implant breaks through the femoral head, a transversal slidability along the longitudinal axis of the pin implant in the guiding structure of the bone plate is further required.

Several solutions are known in the art. One product named Striker© Hansson© Pin System has a pin implant with side protrusions which can be extended from the implant pin when the implant pin is inserted into the femoral neck so that the protrusions are fixated in the femoral head. The protrusions can be deployed by manipulating the pin implant on the proximal end thereof. The Striker Hansson implant pin is fastened on a bone plate having a guiding sleeve to fix the angle between the bone plate and the pin implant.

Another solution is the Synthes © femoral neck system as described in WO 2013/074659 A1. Therein, it is disclosed a bone fixation system comprising an elongated implant shaft including a first channel extending from a proximal end to a side opening formed in a sidewall of the implant shaft along a first channel axis and a bone plate having a first opening to receive a first bone fixation element and the second opening to receive the implant shaft while the first bone fixation element is configured to pass through the side opening of the sidewall of the implant shaft into the head of a bone.

It is an object of the present invention to provide an improved implantation system with a pin implant and a bone plate for the treatment of a bone fracture. Particularly, it is an object of the present invention to provide an improved implantation system for femoral neck fracture fixation. Especially, the implantation system should provide a robust fixation in the bone and should be suitable for absorbing high mechanical loads and torques. It is also desirable to preserve spongiosa during the insertion of the pin implant into the bone.

### Summary of the invention

According to the invention, it is provided an implantation system for the treatment of a bone fracture, particularly for the treatment of a femoral neck fracture, according to claim 1 as well as a handling system for handling an implantation system according to the further independent claim.

Further embodiments are indicated in the subclaims.

According to a first aspect, an implantation system for implantation into a bone of a patient is provided, comprising:
- a longitudinal pin implant for inserting into a first bone part of the bone, wherein the pin implant has a front section with one or more groove-like cut-outs extending in an axial direction of the front section and opening towards the front end of the implant, wherein at least one of the cut-outs is provided as a guidance cut-out which extends over the full length of the pin implant;
- a bone plate including
   ∘ an attachment portion for attachment onto an outer surface of a second bone part, and
   ∘ a guidance sleeve for holding a pin implant and including at least one guidance structure to engage with the at least one guidance cut-outs.

One idea of the above implantation system is to combine the benefits of a pin implant furnished with a cut-out in its front section with a bone plate to provide an implantation system for the treatment of bone fractures. The cut-out may form a groove which at least partially extends along the longitudinal axis of the pin implant. Due to the cut-outs in the front section of the pin implant, the implantation system provides an easy handling since it provides a substantially lower number of handling steps for insertion and fixation in the bone and further provides an improved mechanical stability compared to bone implantation systems of the same kind according to the prior art.

Basically, the implantation system comprises a bone plate for attachment on an outer surface of the second bone part and a pin implant to be inserted in the first bone part in a longitudinal direction thereof. The pin implant may be fastened on the bone plate to provide a fixation of the first and second bone parts, e.g. for the treatment of a bone fracture, by securing the bone parts against a lateral relative movement of the bone parts.

Particularly, the guidance sleeve has a longitudinal axis which protrudes from a main surface of the bone plate so as to fixate a fracture on an angular extension of a bone. The guidance sleeve may be configured to guide the pin implant along a longitudinal axis thereof. So, the pin implant can be inserted and be securely held against tilting with respect to the bone plate. Due to its cut-out-tipped configuration the pin implant can be securely anchored in the first bone part and held therein against a rotation around the longitudinal axis. Thereby, a torque acting via the first bone part on the pin implant is taken up by the fixation of the bone plate on the second bone part. Substantially, a fracture of a femur at the femoral neck can be fixated by implanting the pin implant through the femoral neck into the femoral head and fixating the bone plate on the femoral shaft.

The pin implant can be fully inserted through the guidance sleeve from a proximal end thereof so that in application the bone plate can be readily positioned before the pin implant is inserted into the first bone part. This allows to adapt the position of the bone plate so that a position can be found where the main surface of the bone plate fits best against the outer surface of the second bone part. Once the bone plate has been positioned on the second bone part, the pin implant can be inserted and driven through the second bone part into the first bone part.

The pin implant has a distal front end, a longitudinal shaft section and a proximal rear end. The distal front end incorporates at least one groove-like cut-out which forms a guidance cut-out. This guidance cut-out extends down the longitudinal shaft section, along the full length of the pin implant and opens on the proximal rear end. The guidance cut-out forms the guidance structure which engages with a guiding protrusion on the inner surface of the guidance sleeve of the bone plate. This guidance system allows the rotational orientation of the pin implant to be maintained so that torque applied to the pin implant is directed into the bone plate.

Moreover, the pin implant may have at least one blade structure at the front end directed in axial direction of the pin implant. Such a blade tipped pin implant is easy to be driven into the first bone part so that the stress on and a stress-induced dislocation of the first bone part during insertion is reduced.

It may be provided that the at least one guidance structure of the guidance sleeve has at least one guidance protrusion for engaging with the guidance cut-out of the pin implant so that a single predefined rotational alignment of the pin implant with respect to the guidance sleeve is maintained.

According to an embodiment the pin implant may have a substantially circular cross-sectional profile, wherein the front section is non-threaded, wherein the front section comprises at least three longitudinal groove-like cut-outs extending in axial direction of the front section and opening towards the front end of the pin implant, circumferentially alternating with at least three longitudinal, radially protruding ribs extending in axial direction.

The term "radially protruding ribs" refers to ribs which in the cross-section of the front section of the pin implant protrude over the grooves (cut-outs) in a radial direction relative to the central longitudinal axis of the pin implant. The ribs furthermore extend in a longitudinal axial direction. Thus, the longest extension of the ribs is in the longitudinal direction of the device.

The pin implant of the implantation system has a substantially cross-sectional profile. A "cross-sectional profile", within the meaning of the present invention, is the outline of the projection of the whole body of the pin implant on the plane vertical to the longitudinal axis of the pin implant. Within the meaning of the present invention, "cross-sectional" or the like always refers to a section in a plane vertical to the longitudinal axis of the pin implant, whereas "longitudinal cross-section" or the like refers to a section in the plane of the longitudinal axis of the pin implant. Within the meaning of the present invention, a "substantially circular" profile is a profile that significantly matches a circle with a given radius. In a "substantially circular" profile, the outline may have protrusions or indentions in comparison to a circle. Preferably the outline may have indentions. In specific embodiments, at least 60%, at least 70%, preferably at least 80% and/or more preferably at least 90% of the profile may match a circle with a given radius. Most preferably, the profile may be circular.

The pin implant comprises different sections along its longitudinal axis. The pin implant has a front section, having a front end, a shaft section and a rear end. The pin implant may have several sections between the front section and the shaft section. When implanted into the bone of a subject, the front section may be directed in a proximal direction relatively to the center of the body of the subject and the shaft section may be directed into distal direction. Thus, the terms front/proximal and rear/distal are used interchangeably within the context of the present invention, where technically sensible.

According to the present invention, the pin implant has groove-like cut-outs extending in an axial direction. Within the meaning of the present invention, a "groove-like cut-out" refers to a longitudinally extending indention in the body of the pin implant, irrespective of whether these indentions were obtained by cutting out material from a body or by any other means of manufacture. The cut-outs open towards the front and of the pin implant. Thus, the volume of the cut-out is not confined by material in a lateral direction towards the front end of the pin implant.

The ribs and groove-like cut-outs may alternate circumferentially relative to the central longitudinal axis of the pin implant.

The front part comprising ribs as described alternating with groove-like cut-outs opening towards the front end of the pin implant has a small cross-sectional area. Thus, the volume of spongiosa, which is displaced when the pin implant is driven into the bone, is minimized. Due to the small cross-sectional area, the force required to drive the pin implant into the bone is reduced in comparison to prior art devices. Especially when the pin implant is implanted into a bone, with a groove-like cut-out facing into the direction from which most mechanical force is exerted onto the pin implant, an optimized transfer of mechanical force from the bone on the pin implant is achieved.

The ribs may have an increased cross-sectional width in a section radially more distant to the central longitudinal axis of the pin implant as compared to the width in a section radially closer to the central longitudinal axis of the pin implant.

Furthermore, the ribs may be substantially T-shaped in cross-section.

Within the meaning of the present invention, the "cross-sectional" width of the rib refers to the width of the rib in the plane vertical to the longitudinal axis of the pin implant. At least, the width of a section of at least one rib radially more distant to the longitudinal axis is greater as compared to the width of a second section, radially closer to the longitudinal axis of the pin implant. This definition, within the meaning of the present invention, encompasses ribs which are cross-sectionally substantially T-formed, wherein the base of the "T" is directed towards the longitudinal axis of the pin implant, or which are cross-sectionally substantially L-formed, wherein the top of the "L" is directed towards the longitudinal axis of the pin implant. T-formed ribs are a highly preferred embodiment of the invention. Furthermore, the definition encompasses embodiments, wherein a third section, radially even closer to the central longitudinal axis, as compared to the second section, may also have a greater cross-sectional width as compared to the second section.

The minimized spongiosa displacement comes along with a high mechanical stability, especially a high bending stability of the pin implant. Especially, substantially "T"-formed ribs provide a high bending stability, while requiring few material. Furthermore, although the above-described design of the front part of the pin implant reduces spongiosa displacement upon insertion of the device, the ribbed structure of the front section of the pin implant advantageously provides a high rotational stability of the pin implant in the bone.

In a highly preferred embodiment of the invention, the groove-like cut-outs may be configured radially opposite to the ribs. Preferably, this configuration is achieved by an embodiment of the pin implant, which comprises an uneven number of longitudinal ribs and longitudinal groove-like cut-outs, and wherein the number of longitudinal ribs is the same as the number of longitudinal groove-like cut-outs. The number of longitudinal ribs and longitudinal groove-like cut-outs may each be at least three, at least five and/or at least seven. Preferably, the number of said grooves and said cut-outs is at least three.

In one embodiment the edges at the front end of the pin implant may be configured as cutting edges so that the pin implant is blade tipped.

It may be provided that the protruding end of the ribs comprises convex outer surfaces directed in radial direction. Preferably, in a cross-section profile of the front section, the convex outer surfaces of the ribs all substantially align with a circle, having the longitudinal axis of the pin implant in its center. The outer surfaces of the ribs preferentially extend longitudinally in parallel to the central longitudinal axis of the pin implant. In a further embodiment, the ribs may comprise concave surfaces directed towards the longitudinal groove-like cut-outs. Thus, the concave surfaces of the ribs at least partially define the shape of the groove-like cut-outs. The design of the ribs constituted by convex and concave surfaces advantageously enables the ribs to bear a high mechanical force at a low usage of material.

Moreover, the front end of the convex surface of the ribs may comprise cut-outs configured to decrease the width of the front edges of the ribs and/or wherein the groove-like cut-outs are chamfered towards the front edge of the pin implant.

In a further preferred embodiment of the present invention, the ribs may be configured with an off-set from 100° to 140°, preferably from 110° to 130°, more preferably from 115° to 125°, most preferably of 120°. When the ribs are off-set, for example, by 120°, the angle between the main axis in a radial direction of the two neighboring ribs is 120°.

According to an embodiment one of the groove-like cut-outs may be provided as a non-guidance cut-out which taper off towards the shaft of the pin implant, particularly over the entire length of the groove-like cut-outs.

Furthermore, one of the groove-like cut-outs may be provided as a non-guidance cut-out which comprises a middle section between a front section of the cut-out and a shaft directed end section, wherein the middle section tapers-off linearly towards the shaft and the shaft directed end section tapers-off concavely towards the shaft.

According to an embodiment the pin implant may comprise a cannulation along the longitudinal axis of the pin implant opening to the front and the rear end of the pin implant, wherein particularly a guide wire extends through the guidance sleeve and the cannulation. According to a further embodiment, the diameter of the front section of the pin implant, as defined by the radially protruding ribs, may be smaller than the diameter of the shaft of the pin implant.

The at least one guidance structure of the guidance sleeve may be arranged so that the pin implant is positioned with one of the at least one of the groove-like cut-outs to be directed away from the attachment portion of the bone plate.

Furthermore, the pin implant according to the present invention advantageously provides a maximal surface area in radial projection and therefore minimizes the pressure exerted from the bone structure onto the pin implant. Consequently, mechanical stress on the spongiosa and the pin implant is reduced. The structure of the front section comprising ribs and cut-outs according to the present invention furthermore advantageously increases the total contact area of the pin implant that engages with the bone. Thus, the friction between the pin implant and the bone structure is increased and thus backing out of the pin implant from the bone may advantageously be prevented.

The structure of the pin implant as described herein, especially the structure of the front section, may advantageously prevent the backing out of the pin implant from the bone concomitantly facilitates the insertion into the bone without the need of a thread on the outer surface of the pin implant. Thus, in a preferred embodiment the front section, and/or the intermediate section are non-threaded, especially the outer surface of the front section, and/or the intermediate section and/or the shaft section, or the entire implant may be non-threaded.

As disclosed above, the pin implant may be straight axially inserted without rotation into a variety of different bones. Preferably, the pin implant may be implanted into the femur, preferably with the front part of the pin implant implanted into the femur head and/or neck. Preferably, one groove-like cut-out is directed into a cranial direction upon implantation.

In a preferred embodiment, the groove-like cut-outs may be wider than the ribs. In an embodiment, wherein the groove-like cut-outs are wider than the ribs, the distance between the longitudinal extending edges of the same rib is smaller than the distance between the longitudinal extending edges of two neighboring ribs that constitute a groove-like cut-out.

In a further preferred embodiment of the pin implant according to the present invention, the edges at the front end of the pin implant may be configured as cutting edges, preferably the cutting edges may be configured chisel-like. Thus, the edges at the front end of the pin implant may preferably be configured to minimize the surface area of the pin implant directed into an outward axial direction. The cutting edges at the front end of the pin implant advantageously decrease the forces required for insertion of the pin implant into the bone, especially the spongiosa. Furthermore, the cutting edges reduce bone destruction during insertion of the pin implant. Preferably, the front end of the convex surfaces of the ribs comprises cut-outs configured to decrease the width of the front edges of the ribs. Furthermore, the groove-like cut-outs may be chamfered towards the front edge of the pin implant.

In a preferred embodiment of the pin implant according to the present invention, the groove-like cut-outs comprised in the front section may taper off towards the shaft of the pin implant. This tapering off results in a decreased depth of the groove-like cut-outs from the front end to the end of the groove-like cut-outs directed to the shaft. When the groove-like cut-outs taper off towards the shaft, the distance between the bottom of the groove-like cut-outs and the central longitudinal axis in the longitudinal cross-section of the pin implant increases.

The cut-outs may taper off linearly in at least one section of the cut-outs. The cut-outs may also taper off non-linearly, preferably concavely, in at least one section of the cut-outs. A non-linear tapering of the cut-outs corresponds to a curved shape of the bottom of the cut-out in the longitudinal cross-section of the pin implant. Most preferably, at least the shaft-directed end section of the cut-outs may taper off concavely towards the shaft. The sections tapering off linearly and/or sections tapering off concavely may taper off with a different pitch. The groove-like cut-outs may also comprise several sections that taper off linearly with a different pitch and/or several sections that taper off concavely with a different pitch. The groove-like cut-outs may also comprise at least one section that is untapered. The cut-outs may also comprise sections that taper off linearly and/or sections that taper off concavely and/or sections that are untapered.

Preferably, in a further preferred embodiment, the groove-like cut-outs may taper off towards the shaft over the entire length of the groove-like cut-outs. Within this embodiment, the cut-outs may comprise at least one section that tapers off linearly and at least one section that tapers off non-linearly, preferably concavely.

In a further preferred embodiment of the pin implant according to the present invention, the groove-like cut-outs may comprise at least one middle section between a front section of the cut-outs and a shaft-directed end section, wherein the middle section tapers off linearly towards the shaft and the shaft-directed end section tapers off concavely towards the shaft. Within this embodiment, the middle section and the shaft-directed end section may comprise different sections that taper off with a different pitch.

In another preferred embodiment, the groove-like cut-outs comprise an untapered middle section between a front section of the cut-outs and a shaft-directed tapered end section, wherein the bottom of the cut-outs in said middle section is parallelly aligned with the longitudinal axis of the pin implant in the longitudinal cross-section, and the shaft-directed end section tapers off towards the shaft.

When the pin implant is driven into the bone during the implantation procedure, the tapered sections of the cut-outs advantageously cause a densification of the spongiosa, which leads to an improved stability of the pin implant in the bone.

According to a further embodiment, the pin implant comprises a cannulation along the longitudinal axis of the pin implant, which opens to the front and the rear end of the pin implant. The cannulation may be sized to permit insertion of a guide wire to aid the alignment of the pin implant during the implantation procedure, as commonly known in the art. Additionally, the cannulation provides a possibility to insert / inject a liquid or viscous material through the pin implant. The cannulation may have the same diameter over the whole length of the pin implant or may have different sections along the longitudinal axis of the pin implant with different diameters.

In a further embodiment of the present invention, the bottom of the groove-like cut-outs may comprise at least one opening, preferably several openings, which connect the void of the cut-outs with the cannulation. After insertion of the pin implant into the bone, bone cement or a similar composition that promotes the fixation of the pin implant in the bone may be injected into the cannulation and thereby provided to the bone area surrounding the pin implant through the aforementioned openings. Thus, the openings may advantageously enhance the fixation of the pin implant in the bone.

In a specific further embodiment of the above described embodiment comprising openings connecting the void of the cut-outs with the cannulation, a tube is provided within the cannulation. Furthermore, two strips extending in a longitudinal direction are provided between the outer wall of the tube and the opposite wall of the cannulation. The strips are configured to bend outward through the aforesaid openings upon exertion of a mechanical force on the strips, wherein said force is directed from the rear end of the pin implant towards the front end of the pin implant. The pin implant may furthermore comprise means for fixing the strips in the bended configuration. The bended strips which protrude into the spongiosa may advantageously further restrict a rotational or lateral movement of the pin implant in the bone. A respective design feature is for example discloses in U.S. 2008/0262497.

In a further preferred embodiment of the pin implant, the diameter of the front section of the pin implant may be smaller than the diameter of the shaft section of the pin implant. Within this embodiment, the diameter of the front section may be defined by the radially protruding ribs. A smaller diameter of the front section advantageously facilitates the insertion of the pin implant into a transversal bore of a second implant, such as a bone plate with a guiding sleeve and an intramedullary nail.

In addition to a front section and a shaft section, the pin implant may preferably furthermore comprise an intermediate section between the front section and the shaft section with a diameter tapering from the diameter of the front section to the diameter of the shaft section. The intermediate section may taper off linearly or non-linearly. The intermediate section may also comprise different sections that taper off with a different pitch.

The rear end of the shaft section of the pin implant according to the present invention may be configured and dimensioned for attachment to an insertion device that facilitates the handling of the pin implant during the implantation procedure. The insertion device may for example be an insertion handle or driving cap. The rear end of the shaft section of the pin implant may have a non-symmetrical shape, for example at least one recess, so that the insertion instrument can only be attached in one orientation to the pin implant. Furthermore, the non-symmetrical shape of the rear end of the pin implant prevents a rotation of the pin implant relative to the insertion instrument after attachment of the instrument. Furthermore, the rear end of the shaft may be beveled. The beveled end of the shaft may prevent soft tissue irritation by parts of the rear end of the pin implant protruding from the bone.

In a further embodiment of the invention, the rear end of the cannulation comprises at least one thread. The at least one thread might be configured for attaching an insertion or extraction instrument. Preferably, the rear end of the cannulation comprises two threads, wherein the thread directed to the front of the pin implant has a smaller diameter and the thread directed to the rear end of the pin implant has a larger diameter. Preferably, the two threads run in opposite directions. Preferably, the thread directed to the front end of the device is a left-handed thread, which may be configured to engage with an extraction instrument. Preferably, the thread directed to the rear end of the pin implant is a right-handed thread configured to engage with an insertion instrument. The at least one thread in the rear end of the pin implant is configured in a bore or cannulation. Thus, the at least one thread is preferably not configured on the outer surface of the pin implant.

Moreover, the attachment portion of the bone plate may extend from a proximal end of the guidance sleeve in at least one direction thereof, wherein particularly the axial direction of the guidance sleeve and a longitudinal direction of the attachment portion encompass an obtuse angle therebetween.

It may be provided that the guidance sleeve has a guidance structure formed as a guidance protrusion at a distal end in the interior of the guidance sleeve.

According to a further aspect, a handling tool for an implantation system particularly for the above implantation system, is provided, wherein the handling tool comprises:
- a tool sleeve portion with a longitudinal sleeve;
- a connector element slidable along the axis of the longitudinal sleeve, wherein the connector element includes a receiving portion configured to receive an attachment portion of a bone plate along an insert direction and to hold the connector element with respect to any other direction;
wherein the tool sleeve portion has a locking means for engaging with the attachment portion in an axial direction of the tool sleeve portion, when the connector element is moved along the axial direction of the tool sleeve portion, wherein the insert direction and the axial direction are inclined to each other so that the bone plate is locked after insertion in the receiving portion followed by an engagement of the locking means with the attachment portion in the axial direction.

Furthermore, the locking means may be formed at a holding end of the longitudinal sleeve. According to an embodiment a handling system with the above handling tool and a bone plate of an implantation system may be provided, wherein the bone plate has an attachment portion with indentations to engage with holding protrusions of a recess of the receiving portion of the connector element.

In a further aspect, the present invention relates to the use of the pin implant according to the present invention as described above, for treating bone fractures, preferably fractures of a long bone, more preferably a fracture of the humerus or the femur, most preferably fractures of the femur. Accordingly, the present invention also relates to the use of the implant according to the present invention for implantation into a bone, preferably into a long bone, more preferably into the humerus or the femur, most preferably into the femur.

In still another aspect, the present invention relates to a method of surgery or method of treating bone fractures, wherein said method comprises at least the step of implanting an implant according to the present invention into a bone.

### Brief description of the drawings

Embodiments are described in more detail in conjunction with the accompanying drawings, in which:
- Figure 1: shows a side view onto an implantation system;
- Figure 2: shows a side view onto the implantation system inserted into a fractured femur of a patient;
- Figure 3: shows a side view onto a bone plate of the implantation system;
- Figure 4: shows a cross-sectional side view onto the bone plate of the implantation system;
- Figure 5: shows a lateral side view onto the bone plate of the implantation system;
- Figure 6: shows a perspective view onto the bone plate of the implantation system;
- Figure 7: shows another perspective view onto the bone plate of the implantation system;
- Figure 8: shows a bottom side view onto the bone plate of the implantation system;
- Figure 9: shows a cross-sectional view onto the guidance sleeve of the bone plate of the implantation system;
- Figure 10: shows an exemplary pin implant according to an embodiment in perspective view partially from the front;
- Figure 11: shows the pin implant in a perspective view, partially from the rear;
- Figure 12: shows a front section of the pin implant in a perspective view;
- Figure 13: shows a front view of the pin implant;
- Figure 14: shows a cross-sectional view through a front part of the pin implant comprising ribs and cut-outs;
- Figure 15: shows a longitudinal cross section through the front part of the pin implant comprising a groove-like cut-out with an tapered section along X - X;
- Figure 16: shows a longitudinal section through the entire pin implant, wherein the groove-like cut-outs comprise openings.
- Figure 17: show a flow chart illustrating the steps for implanting the implantation device into a bone;
- Figure 18: shows a partially cross-sectional perspective view onto the handling tool with a tool sleeve portion and a connector element;
- Figure 19: shows a cross-sectional view onto the connector element;
- Figure 20: shows a lateral view onto the connector element;
- Figure 21: shows a cross-sectional view onto the tool sleeve portion;
- Figure 22: shows a lateral view onto the handling end of the tool sleeve portion;
- Figure 23: shows a partially cross-sectional view of the handling tool before mounting a bone plate;
- Figure 24: shows a partially cross-sectional view of the handling tool with the bone plate inserted into the receiving portion;
- Figure 25: shows a partially cross-sectional view of the handling tool with the bone plate interlocked in the receiving portion of the connector element;
- Figure 26: shows a cross-sectional view through of the handling tool across an axial direction E of the tool sleeve portion with the bone plate hold in the receiving portion of the connector element and being locked;
- Figure 27: shows a cross-sectional view through of the handling tool across an insert direction D of the connector element with the bone plate hold in the receiving portion of the connector element and being locked;
- Figure 28: shows a perspective view onto the connector element into the receiving portion; and
- Figure 29: shows a perspective view onto the holding end of the tool sleeve portion.

### Description of embodiments

The implantation system according to the present invention relates to the treatment of bone fractures wherein a first and a second bone part are secured in a fixed position relative to each other which is capable of bearing a significant load. Particularly, the following description of the embodiments is made with respect to the fixation of femoral neck fractures. However, although the following description describes the present invention with respect to the treatment of a fracture of the femoral neck, the implantation system according to the present invention can be applied to any other bone in the body.

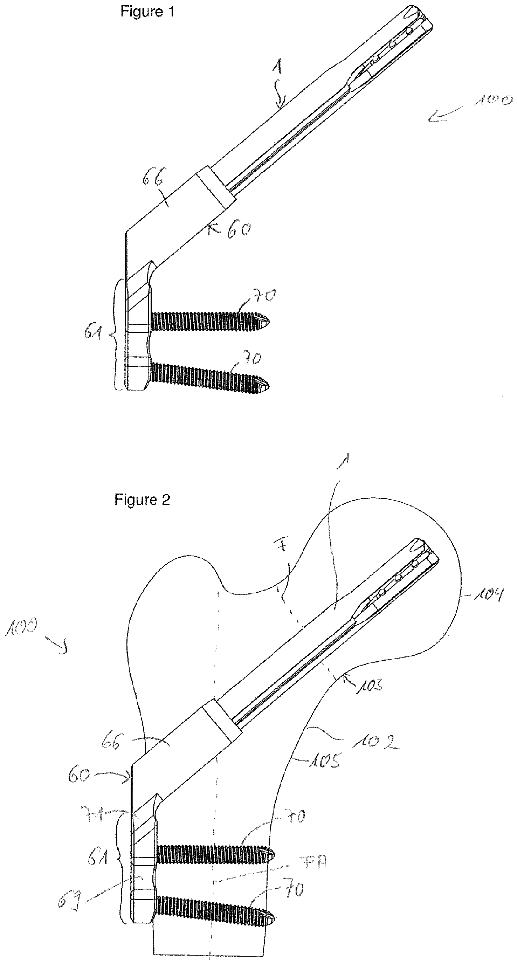

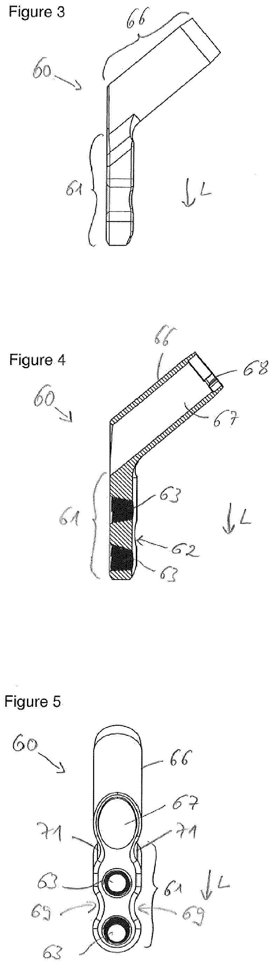

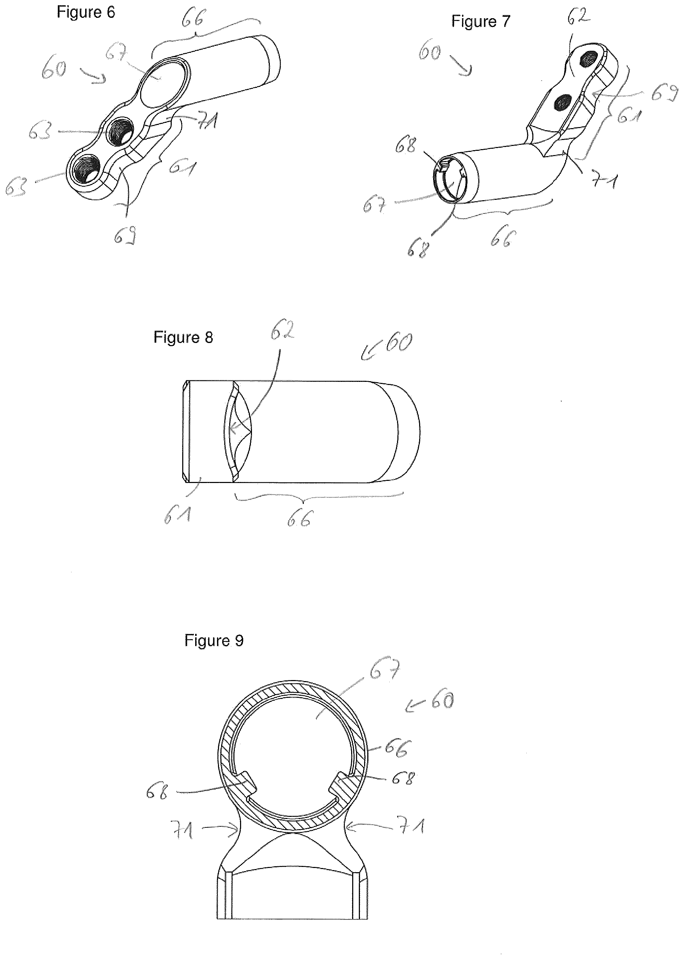

The implantation system is described in detail in conjunction with the drawings of Figs 1 to 16.

The implantation system 100 comprises a pin implant 1 and a bone plate 60 for fixation of a fracture F of a femoral neck 103 of a femur 102 of a patient. The pin implant 1 is to be inserted through the femoral neck 103 into the femoral head 104 (first bone part). The bone plate 60 is configured to be attached on a target surface portion of a femoral shaft 105 (second bone part) of the femur 102 opposing the femoral head 104. The fracture F of the femur separates the femoral shaft 105 (first bone part) and the femoral head 104 (first bone part).

The bone plate 60 comprises an attachment portion 61 having a first main surface 62 for engagement with the target surface portion of the femoral shaft 105 of the femur 102. The attachment portion 61 may have may be elongated in a direction L to be applied on the femoral shaft 105 so that the longitudinal axis of the attachment portion 61 is substantially parallel to a longitudinal axis FA of the femoral shaft 105.

The attachment portion 61 may be provided with one or more through holes 63 for receiving bone fixation elements 70 to attach the attachment portion 61 of the bone plate 60 with its first main surface 62 at the femoral shaft 105. The through holes 63 may be arranged distanced in the attachment portion 61 preferably along the longitudinal direction L thereof.

The fixation of the attachment portion 61 at the femoral shaft may be made with one or more bone fixation elements 70 to be inserted into the femoral shaft 105. The through holes 63 may be provided as screw holes having a thread, which may be conical in a preferred embodiment. The screw holes may be threaded to engage with a complimentary thread of a bone screw as a bone fixation element 70. So, if the bone screw has been inserted into the femoral shaft 105, the thread of the screw hole and the complimentary thread of the bone screw engage and secure the bone screw on the attachment portion against a tilting movement of the bone plate 60. So, all torques and forces applied on the bone plate 60 are directed to the femoral shaft 105 via the attachment portion 61 and the bone fixation elements 70.

The screw axis of the through holes 63 may extend perpendicular to the first main surface 62 of the attachment portion 61, however the screw axis of the through holes 63 can be tilted to include an angle with the first main surface 62 of the attachment portion 61 of between 70° to 90° with respect to the longitudinal direction L of the attachment portion 61 or with respect to a direction Q perpendicular to the longitudinal direction L of the attachment portion 61. Preferably, the axes of the through holes 63 extend into different directions.

The bone plate 60 has a sleeve portion 66 which includes a guidance sleeve for receiving the pin implant 1. The guidance sleeve has a cylindrical through opening 67, preferably with a substantially circular cross-section to receive the pin implant 1 with a corresponding substantially circular cross-section. The guidance sleeve extends away from the first main surface 62 of the attachment portion 61 angled between 90° and 170°. For the fixation of a femoral neck fracture, the preferred angle is between 115° and 145°, preferably approximately 130°, which substantially corresponds to the angle between a femoral shaft and a femoral neck. Other angles may cope with the anatomy of the patient or cope with a bone fracture of other bones than the femur.

The sleeve portion 66 may extend from one end of the attachment portion 61 preferably from an end with respect to the longitudinal direction thereof. The sleeve portion 66 extends only on one side from the first main surface of the attachment portion 61 leaving a second main surface 67 of the attachment portion 61 substantially plain or slightly curved.

In conjunction with the exemplary embodiment of the pin implant 1, the pin implant 1 is provided which has a circular cross-section which substantially corresponds to the circular cross section of the inner through opening 67 of the guidance sleeve 66 to be inserted thereon. Substantially, the pin implant 1 is adapted to be slidably held in the through opening 67 of the sleeve portion 66 so that basically the pin implant 1 can be inserted into the through opening 67 from the side of the second main surface of the attachment portion 61 and inserted into the bone through the second bone part into the first bone part, i.e. in direction of the femoral head through the sleeve portion 66.

In its end position, the pin implant 1 is held in the femoral head with its front end, while a part of a shaft portion of the pin implant 1 is positioned in the through opening 67 of the sleeve portion 66. After implantation, the rear end of the pin implant 1 is preferably positioned within the through opening 67 of the sleeve portion 66. Preferably, the rear end is positioned that it does not protrude over the second main surface of the attachment portion 61. The sleeve portion 62 substantially has a length in an axial direction which allows to take up any torque around an axis perpendicular to the sleeve axis.

Furthermore, the pin implant 1 may be provided with a groove-like guidance cut-out extending from the front end 11 to the rear end 31 thereof, which in cross-section may form a recess with respect to the preferably circular profile of the pin implant 1. Basically, the guidance cut-out may be a groove which engages with a guidance structure, such as a guidance protrusion 68, within the through opening 67 of the sleeve portion 66 so that the rotational position of the inserted pin implant 1 is predefined with respect to the position of the bone plate 60.

The guidance cut-out and the guidance protrusion 68 within the sleeve portion 62 allow to secure the pin implant 1 against a rotational movement, so that when the front end of the pin implant 1 is securely attached in the femoral head, the femoral head is securely fixed with respect to the bone shaft against a rotational movement.

Substantially, a pin implant 1 may have at its front end 11 a rib structure with one or more blades directed into the longitudinal direction of the pin implant 1 so that the pin implant 1 can be easily driven into the femoral neck and femoral head by applying a transversal force into its longitudinal direction. This allows to decrease the separation force acting onto the fracture F during the implantation process.

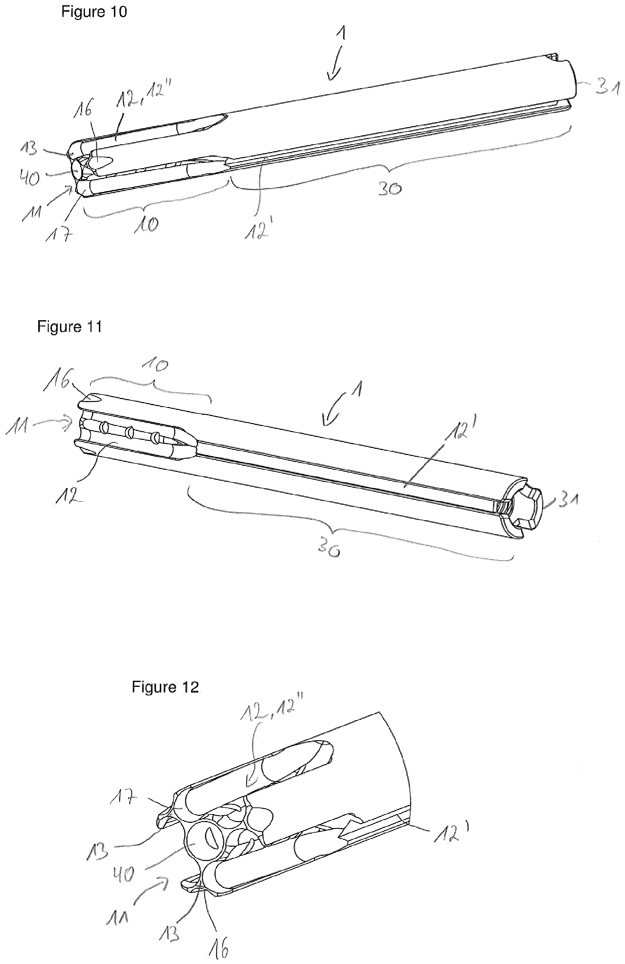

In detail Figures 10 to 16 show an example of a longitudinal pin implant 1 with a substantially circular cross-section profile is provided, comprising a front section 10 with a front end 11, a shaft section 30 and a rear end 31. The front section 10 may comprise at least three longitudinal groove-like cut-outs 12 extending in the axial direction of the front section 10 and opening towards the front end 11 of the pin implant 1, circumferentially alternating with at least three longitudinal protruding ribs 13 extending in an axial direction is shown. The embodiments exemplified in the figures 10 to 16 may all have a configuration wherein the groove-like cut-outs 12 are configured radially opposite to the ribs 13.

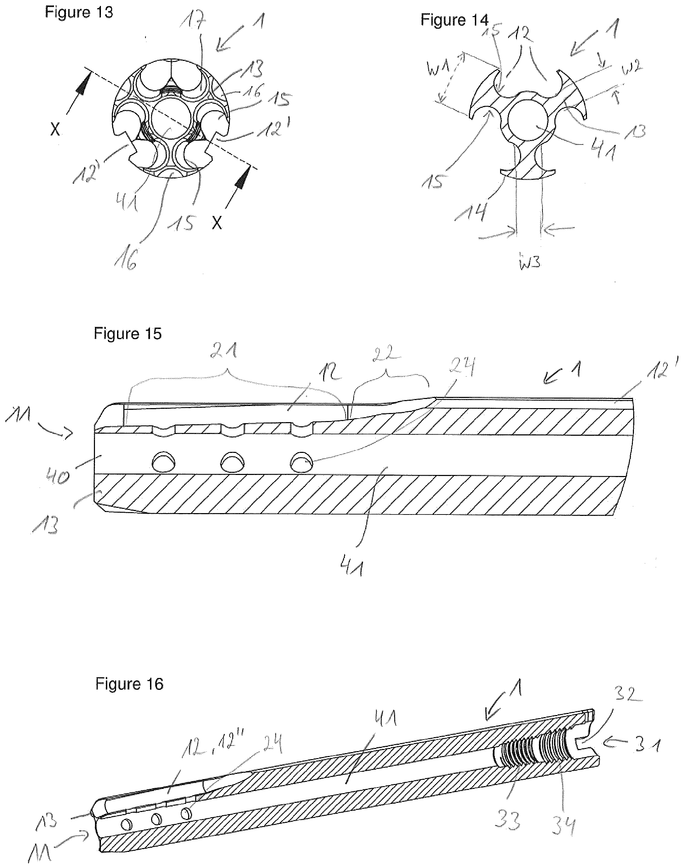

The substantially circular cross-sectional profile of the pin implant 1 corresponds to the view along the longitudinal axis of the pin implant 1, which is for example depicted in Fig. 13.

As shown in Fig. 14, the ribs 13 in the front section 10 of the pin implant 1 may have an increased cross-sectional width (W1) in a section radially more distant to the central longitudinal axis of the implant, as compared to the width (W2) in a section radially closer to the central longitudinal axis of the implant. Furthermore, the ribs 13 may comprise a third section, radially even closer to the central longitudinal axis, as compared to the second section, which may have a greater cross-sectional width (W3) as compared to the second section. The embodiment shown in Fig. 14 may encompass ribs which are substantially T-formed with respect to the cross-section, wherein the base of the "T" is directed towards the longitudinal axis 40 of the implant.

In the pin implant of Figures 10 to 16, the protruding ends of the ribs 13, thus the ends of the ribs 13 opposite to the longitudinal axis of the pin implant 1, may comprise convex outer surfaces 14 directed in the radial direction. The convex outer surfaces 14 of the ribs 13 may all substantially align with a circle having the longitudinal axis 40 of the implant in its center, which is, for example, schematically shown in Figures 13 and 14. Furthermore, the ribs 13 may comprise concave surfaces 15 directed towards the longitudinal groove-like cut-outs.

In a preferred embodiment of the pin implant 1, the edges at the front end 10 of the pin implant 1 may be configured to minimize the surface area of the pin implant 1 directed into outward axial direction. Therefore, as shown in Figure 12, the front end of the convex surfaces of the ribs 13 may comprise cut-outs 16 configured to decrease the width of the front edges of the ribs 13 or tapering towards the front end of the pin implant 1. Furthermore, the groove-like cut-outs 12 may comprise chamfered sections 17 towards the front edge of the implant.

At least one of the groove-like cut-outs 12 is formed as a guidance cut-out 12' extending over the full length of the pin implant. At least one of the other groove-like cut-outs 12 which is formed as a non-guidance cut-out 12" and is comprised in the front section 10 may taper off towards the shaft section 30 of the pin implant 1, as, for example, shown in Figure 15. When the non-guidance groove-like cut-outs 12" taper off towards the shaft section, the distance D1 in the longitudinal cross-section of the implant between the bottom of the groove-like cut-outs and the central longitudinal axis 40 increases. The pin implant 1 shown in Figure 15 exhibits cut-outs (recession) 14 within a section 21 that tapers off linearly and a section 22 that tapers off concavely.

In the embodiments shown in Figs. 10 to 16, the implant according to the invention comprises a cannulation 41 along the longitudinal axis 40 of the implant opening to the front 11 and the rear end 31 of the implant. In the embodiment of the implant shown in Figure 16, the bottom of the groove-like cut-outs 12 comprises openings 42 which connect the void of the cut-outs with the cannulation 41.

As shown, for example, in Figure 11, the rear end 31 of the shaft section 30 of the implant may be configured and dimensioned for attachment of an insertion device that facilitates the handling of the implant during the implantation procedure. For example, the rear end 31 of the shaft section may have a recess 32. The embodiments shown in Figures 11 and 16 furthermore comprise two threads 33, 34, wherein the thread 33 directed to the front end of the implant has a smaller diameter when the thread 34 directed to the rear end 31 of the implant. The two threads may run in opposite directions.

The front section of the pin implant 1 comprising ribs alternating with groove-like cut-outs has a minimal cross-sectional area. Thus, when the front end of the pin implant 1 is driven into a bone, the volume of a spongiosa which is displaced by the pin implant is minimized. This minimized spongiosa displacement is advantageously combined with a high stability, especially bending stability, of the ribs in the pin implant 1 of the present invention. Furthermore, the forces for insertion of the pin implant 1 into the bone are reduced. Advantageously, the pin implant 1 can be implanted into a bone with a groove-like cut-out 12 facing into the direction from which the highest mechanical force is exerted onto the implant. Thereby, only little spongiosa is displaced in the direction from which the force is applied, which enables an optimized transfer of the mechanical force from the bone to the pin implant 1.

When the pin implant 1 is used for the treatment of femur fractures, one groove-like cut-out 12 may be directed in the cranial direction, which advantageously preserves spongiosa material in the area that bears the highest mechanical forces, and thus advantageously enables optimized force distribution and bone preservation.

Especially the substantially T-formed ribs provide a high bending stability while requiring only little material. Furthermore, although the above-described design of the front part of the implant reduces spongiosa displacement upon insertion of the device into the bone, the ribbed structure of the front section 10 of the pin implant 1 advantageously provides a high rotational stability of the pin implant 1 in the bone.

The pin implant 1 advantageously provides a maximal support area in radial projection and therefore generally minimizes the pressure exerted from the bone structure onto the implant and thus relieves the spongiosa and the implant. The structure of the front section 10 comprising ribs and cut-outs according to the present invention, furthermore advantageously increases the total contact area of the implant that engages with the bone. Thus, the friction between the pin implant 1 and the bone structure is increased and consequently the backing out of the pin implant 1 from the bone is advantageously prevented. In addition, the tapered sections of the cut-outs advantageously cause a densification of the spongiosa, which leads to an improved stability of the implant in the bone.

When comparing the stability of a pin implant according to the present invention, comprising a front section with groove like cut-outs, with state of the art helical blade implants, it was found that implant according to the invention exhibited a higher stability in an artificial bone model. While exertion of high forces on pin implants inserted into a cellular rigid polyurethane foam only induced a slight migration, the same force induced a cutting-out of the helical blades from the test material.

Furthermore, the cutting edges at the front of the implant advantageously decrease the forces required for insertion of the pin implant 1 into the bone.

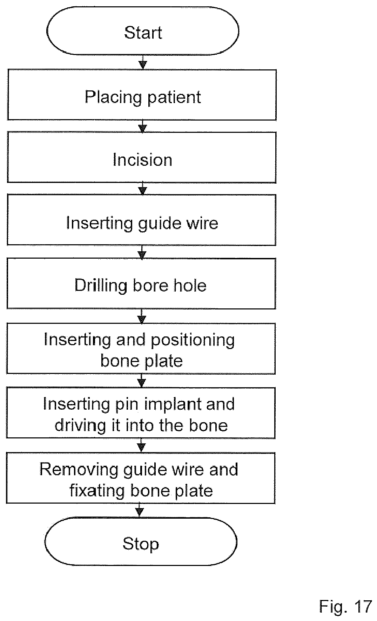

In Figure 17, a flow chart of an exemplary method of use of the implantation system is shown. In a first step S1, the patient is placed in a preferred, e.g. supine, position, and the bone parts of the fractured bone are brought into a corrected alignment.

In steps S2, an incision is made in a longitudinal direction in proximity to the greater trochanter of the femur. The femoral shaft of the femur is then exposed to gain access to the bone.

As a next step S3, a guide wire is positioned and inserted into the bone along a path which shall define the extension of a bore hole to be made. The depth by which the guide wire is inserted is set that a distal end of the guide wire is fixed in the femur head. In step S4, a bore hole is made on the exposed origin of the femoral neck into the femur head along the path defined by the guide wire.

The bore hole is made with a depth into the femur head which allows a good insertion of the sleeve portion and a robust fixation of the pin implant. The pin implant is selected with a length corresponding to the insertion depth of the guide wire.

Particularly, the bore hole may be drilled with a first diameter and with a depth which corresponds to the length of the sleeve portion, i.e. the protrusion length from the first main surface 62 of the attachment portion 61. The pin implant 1 may be provided with a substantial longer length and is driven into the bone material of the femur neck and the femur head. To facilitate the insertion of the pin implant 1 a deeper bore hole with a second smaller diameter can be provided through the femoral neck into the femoral head with a length substantially corresponding to the length of the pin implant to be inserted.

In step S5, the bone plate 60 is guided over the guide wire and inserted with its sleeve portion into the bore hole. The bone plate 60 can then be rotated around the sleeve portion inserted into the bore hole to find a best position for the fixation of the attachment portion 61 onto the bone shaft (second bone part). This is made by rotating the bone plate 60 around the sleeve portion which is held in the bore hole when a good position for fixation has been found. Depending on the tolerance between the borehole's diameter and the outer diameter of the sleeve portion, the bone plate 60 can be slightly tilted within a limited range to provide a good attachment of the attachment portion 61 on the outer surface of the bone shaft.

In step S6, the pin implant 1 is then guided over the guide wire with its cannulation and inserted on a proximal end of the sleeve portion 66. The pin implant 1 can be driven into the femoral neck and femoral head through the through opening 67 of the sleeve portion 66 in a direction as determined by the axial direction of the through opening 67. The insertion of the pin implant may be made by an impactor, wherein the front end of the pin implant 1 is driven into the femoral head until the rear end of the pin implant 1 is substantially flush to the second main surface of the attachment portion 61 of the bone plate 60. The front end of the pin implant 1 then cuts through the bone material (cancellous bone), compacts the bone material and provides a secure and stable fixation of the pin implant in the bone material of the femoral neck.

The insertion of the pin implant 1 is made so that the guidance cut-out 12' along the axis of the pin implant engages with the corresponding guidance structure in the through opening 67 of the sleeve portion 66 so that the rotational position of the pin implant 1 is fixed and the groove like cut-out is directed upwards, i.e. in a direction defined by the direction of arrangement of the attachment portion and guidance sleeve of the bone plate 60.

After the pin implant has been inserted, in step S7 the guide wire is removed and the bone plate 60 is fixated on the second bone part (femoral shaft) by bone fixation elements, such as the bone screws. In another embodiment, the bone plate 60 may be fixated at the bone before the pin implant 1 is driven into the femoral neck of the bone, until the rear end 31 of the pin implant 1 leads flush against the bone. The guide wire is now removed from the bone, and the handling device may be removed from the bone plate 60 leading the implantation system 100 in the bone.

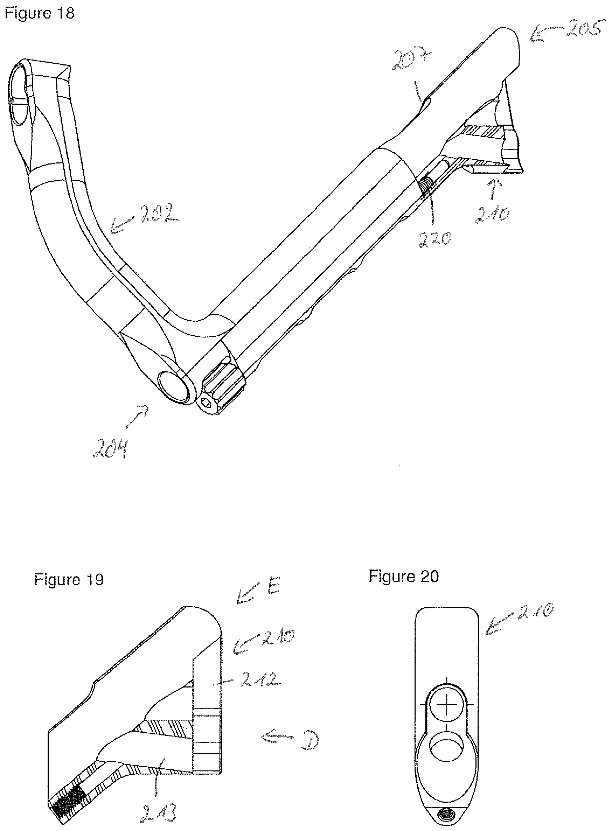

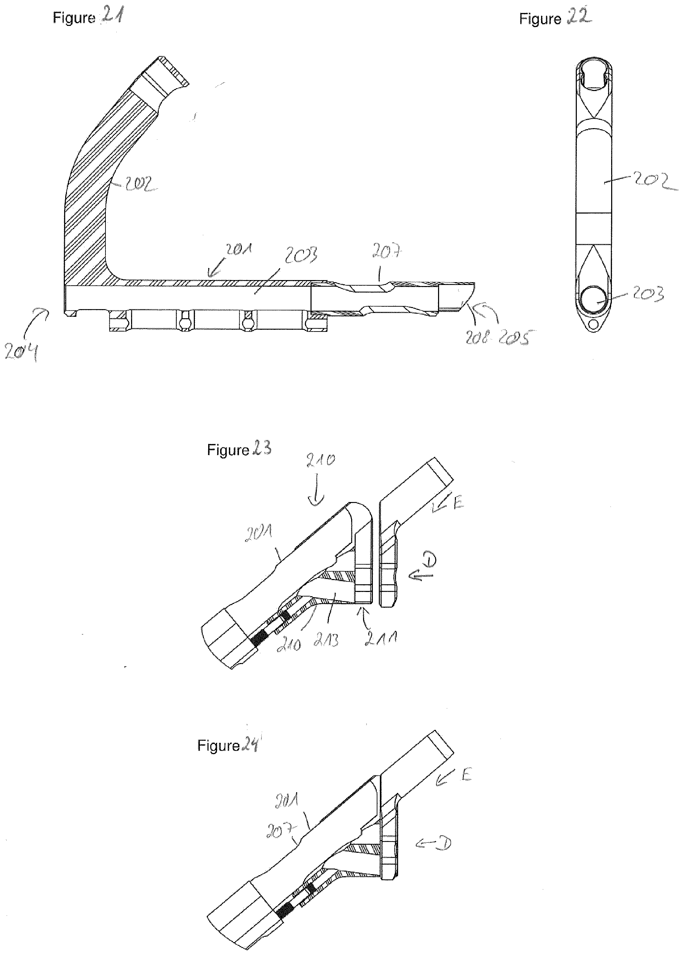

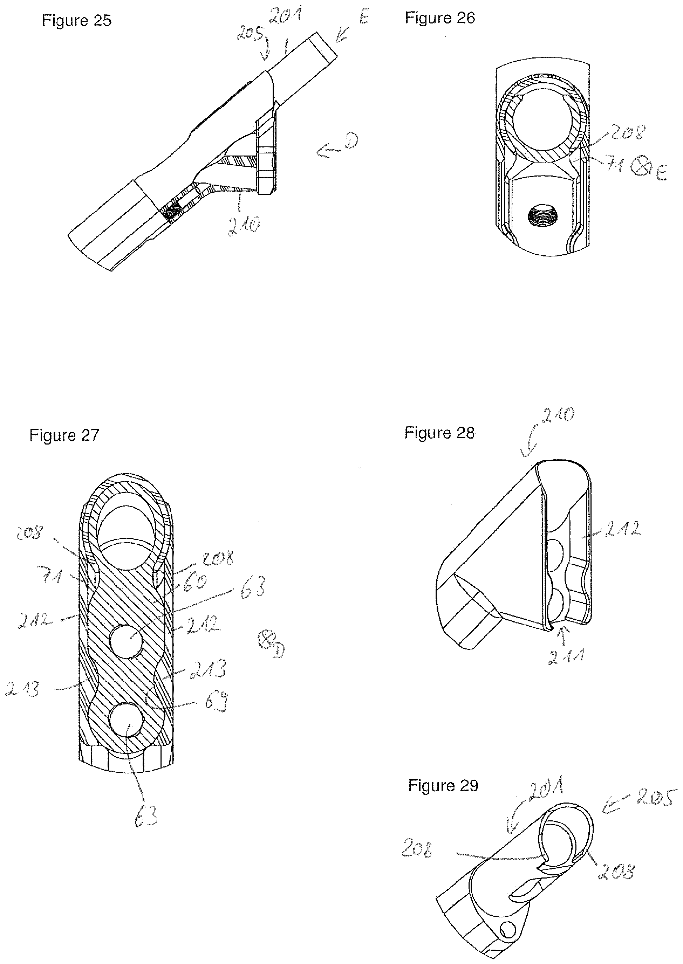

Figures 18 to 26 show a handling tool 200 and its parts for supporting the implantation of the implantation system 100 into the bone. The handling tool 200 allows to hold the bone plate 60 during the implantation process and allows to position the bone plate and to insert the pin implant 1 over the guide wire.

The handling tool 200 has an elongated tool sleeve portion 201 and an arm portion 202 protruding from an axis of the sleeve portion 201. The elongated tool sleeve portion 201 has an inner substantially cylindrical through hole 203 through which the drill for drilling the bore hole into the bone may be inserted, through which the guide wire is guided and attached in the bone and through which the pin implant 1 may be driven into the bone.

The arm portion 202 extends from the tool sleeve portion 201 and facilitates handling of the handling tool 200 for manipulating the bone plate 60. The arm portion 202 is depicted with a curvature, however, any other shape may be used to allow an easy handling of the implantation system 100, particularly by applying a torque around the longitudinal axis of the tool sleeve portion 201. The handling tool 200 has a handling end 204 close to which the arm portion 202 is located and a holding end 205 where the bone plate 60 can be releasably attached for manipulation of its position.

The holding end 205 is provided with a connector element 210 which is slidably movable along the tool sleeve portion 201 and which provides a receiving portion 211 to receive the bone plate 60 which its attachment portion. The receiving portion 211 is directed towards the holding end 205 and may be formed with a recess having an inner contour/shape corresponding to the outer contour/shape of the attachment portion 61 of the bone plate 60.

The receiving portion 211 is configured to allow the attachment portion 61 of the bone plate 60 to be slid thereon along an insert direction D. Then, the bone plate is received in the recess of the receiving portion 211 so that it is held against any movement other than the movement into the insert direction D. The insert direction D is the direction in which the bone plate 60 is inserted by sliding it into the receiving portion 211. For fixation, the bone plate 60 in the receiving portion 211 may be hold against a movement in all directions but the insert direction D. In the present embodiment, the edges 212 (walls) protruding into the insert direction D form a recess in which the attachment portion is accommodated. By means of opposing holding protrusions 213 extending along the insert direction D and protruding from the edges 212 into the recess of the receiving portion 211 contribute to the fixation of the bone plate 60. The bone plate 60 is provided with first indentations grooves 69 extending across the width of the attachment portion 61 in an insert direction D perpendicular to the longitudinal direction L of the attachment portion 61. The indentations grooves 69 serve as a first sliding guidance for the bone plate 60 into the connector element 210.

Furthermore, the attachment portion 61 of the bone plate 60 has fixation means e.g. formed as second indentations grooves 71 which extends into the axial direction E of the sleeve portion 66. The second indentations grooves 71 serve as a second sliding guidance which allow a locking of the bone plate 60. The axial direction of the sleeve portion 66 and the direction E are non parallel to each other.

The tool sleeve portion 201 has at its holding end two locking legs 208 which may engage with the second indentations grooves 71 of the bone plate 60 received in the receiving portion 211. Engagement is made when the connector element 210 is moved together with the bone plate 60 along the axial direction towards the handling end 204 or when the tool sleeve portion is moved towards its holding end 205 so that the locking legs 208 engage through the second indentations grooves 71. The inclination between the insert direction D and the direction E then locks the bone plate 60 onto the handling tool 200, so that a manipulation of the bone plate 60 when inserted into the bore hole can be easily made.

Furthermore, by moving the connector element 210 towards the holding end 205 the bone plate 60 can be released without a relative movement between the bone plate 60 and the handling tool 200 across its axial direction. This allows to keep the space requirement for the implantation process low as only little lateral movement is necessary to release the bone plate from the handling tool 200.

The inclination between the insert direction D and the axial direction E is substantially defined by the angle between the sleeve portion 66 and the longitudinal direction L of the attachment portion 61 of the bone plate 60 which is approximately 130°. Substantially for the locking mechanism of the handling tool 200 it is required that the angle is more than 100° to provide a secure fixation of the bone plate 60 at the handling tool 200.

In general, the locking of the bone plate 60 uses a first sliding guidance provided between the connector element 210 and the bone plate 60 and a second guidance provided between the tool sleeve portion 201 and the bone plate 60. The locking is made by a relative movement of the tool sleeve portion 201 and the connector element 210.

The connector element 210 may be hold in its locking position by means of a securing screw 220 which may be arranged coaxially with the longitudinal axis of the tool sleeve portion. The connector element 210 may be provided with a thread into which the securing screw 220 may engage. So by turning the securing screw 220, the connector element 210 is moved in axial direction of the tool sleeve portion 201. When the connector element 210 is shifted towards the holding end of the tool sleeve portion the bone plate is secured onto the handling tool 200.

Furthermore, the tool sleeve portion 201 has a through hole 207 across its longitudinal direction which allows access to the through holes 63 of the attachment portion 61 of the bone plate 60 when the bone plate 60 is locked onto the handling tool 200 so that the bone fixation elements 70 can be attached and inserted into the bone through the through hole 207 in the tool sleeve portion 201. Correspondingly, the connector element 210 which can be slidably moved along the tool sleeve portion 201 has an through opening 213 for each through hole of the bone plate 60, wherein in the locking position in which the bone plate 60 is locked onto the holding end of the tool sleeve portion 201, the through hole 207 and the opening(s) 213 are aligned to each other thereby allowing the insertion of the bone fixation elements through the aligned openings.

The handling arm 200 can be provided with an alignment opening, which allows to insert a screw fixation tool so that the alignment of the bone fixation element is defined by the line between the alignment opening in the arm portion, a through opening through the tool shaft and the fixation holes in the attachment portion of the bone plate. Using two or more fixation openings in the attachment portion together with a single alignment opening in the handling arm allows to insert the bone fixation elements in a non-parallel relation to each other thereby providing a more reliable fixation of the bone plate 60 onto the bone shaft.

## Claims

1. Implantation system for implantation into a bone of a patient, comprising:
- a longitudinal pin implant for inserting into a first bone part of the bone, wherein the pin implant has a front section with one or more groove-like cut-outs extending in axial direction of the front section and opening towards the front end of the implant, wherein at least one of the cut-outs is provided as a guidance cut-out which extends over the full length of the pin implant;
- a bone plate including
∘ an attachment portion for attachment onto an outer surface of a second bone part, and
∘ a guidance sleeve for slidably holding the pin implant and including at least one guidance structure to engage with the at least one guidance cut-outs.

2. Implantation system according to claim 1, wherein the pin implant has at least one blade structure at the front end directed in axial direction of the pin implant so that edges at the front end of the pin implant are configured as cutting edges.

3. Implantation system according to claim 1 or 2, wherein the at least one guidance structure of the guidance sleeve has at least one guidance protrusion for engaging with the guidance cut-out of the pin implant so that a predefined rotational alignment of the pin implant with respect to the guidance sleeve is provided.

4. Implantation system according to any of the claims 1 to 3, wherein the pin implant has a substantially circular cross-sectional profile, wherein the front section is non-threaded, wherein particularly the front section comprises at least three longitudinal groove-like cut-outs extending in axial direction of the front section and opening towards the front end of the implant, circumferentially alternating with at least three longitudinal, radially protruding ribs extending in axial direction.

5. Implantation system according to claim 4, wherein the ribs have an increased cross-sectional width in a section radially more distant to the central longitudinal axis of the pin implant as compared to the width in a section radially closer to the central longitudinal axis of the pin implant.

6. The implantation system of any of claims 4 to 5, wherein the protruding end of the ribs comprises convex outer surfaces directed in radial direction and/or the ribs comprise concave surfaces directed towards the longitudinal groove-like cut-outs.

7. The implantation system of any of claims 4 to 6, wherein one of the groove-like cut-outs is provided as a non-guidance cut-out which taper off towards the shaft of the implant, particularly over the entire length of the groove-like cut-outs.

8. The implantation system of any of claims 1 to 7, wherein the implant comprises a cannulation along the longitudinal axis of the pin implant opening to the front and the rear end of the pin implant, wherein particularly a guide wire extends through the guidance sleeve and the cannulation.

9. The implantation system of any of claims 1 to 8, wherein the at least one guidance structure of the guidance sleeve is arranged so that the pin implant is positioned with one of the at least one of the groove-like cut-outs to be directed away from the attachment portion of the bone plate.

10. The implantation system of any of claims 1 to 9, wherein the attachment portion of the bone plate extends from a proximal end of the guidance sleeve in at least one direction thereof, wherein particularly the axial direction of the guidance sleeve and a longitudinal direction of the attachment portion encompass an obtuse angle therebetween.

11. The implantation system of any of claims 1 to 10, wherein the guidance sleeve has a guidance structure formed as a guidance protrusion complementary to the guidance cut-out at a distal end in the interior of the guidance sleeve.

12. Handling tool for an implantation system particularly for an implantation system according to any of the claims 1 to 11, wherein the handling tool comprises:
- a tool sleeve portion with a longitudinal sleeve;
- a connector element slidable along the axis of the longitudinal sleeve, wherein the connector element includes a receiving portion configured to receive an attachment portion of a bone plate along an insert direction and to hold the connector element with respect to any other direction;
wherein the tool sleeve portion has a locking means for engaging with the attachment portion in an axial direction of the tool sleeve portion, when the connector element is moved along the axial direction of the tool sleeve portion, wherein the insert direction and the axial direction are inclined to each other so that the bone plate is locked after insertion in the receiving portion followed by an engagement of the locking means with the attachment portion in the axial direction.

13. Handling tool according to claim 12, wherein the locking means are formed at a holding end of the longitudinal sleeve.

14. Handling system with a handling tool according to claim 12 or 13 and a bone plate of an implantation system, wherein the bone plate has an attachment portion with indentations to engage with holding protrusions of a recess of the receiving portion of the connector element.
